# EUROPÄISCHE PATENTSCHRIFT

(11) **EP 1 592 663 B1**
(45) Veröffentlichungstag und Bekanntmachung des Hinweises auf die Patenterteilung: **26.06.2013**
(21) Anmeldenummer: 04705474.7
(22) Anmeldetag: 27.01.2004
(51) Int. Cl.: C07D 211/34

(54) **VERFAHREN ZUR HERSTELLUNG VON D-THREO-2-PHENYL-2-PIPERIDIN-2-YL-ESSIGS UREESTERN**
METHOD FOR THE PRODUCTION OF D-THREO-2-PHENYL-2-PIPERIDINE-2-YL ACETATES
PROCEDE DE FABRICATION D'ESTERS D'ACIDE D-THREO-2-PHENYL-2-PIPERIDINE-2-YL-ACETIQUE

(30) Priorität: 05.02.2003 CH 163032003
(43) Veröffentlichungstag der Anmeldung: 09.11.2005
(73) Patentinhaber: Siegfried AG, 4800 Zofingen (CH)
(72) Erfinder: TRAFELET, Huldreich, CH-4802 Strengelbach (CH); WOLLENWEBER, Markus, CH-4800 Zofingen (CH)
(74) Vertreter: Braun, André jr.
(86) Internationale Anmeldenummer: PCT/IB2004/000265
(87) Internationale Veröffentlichungsnummer: WO 2004/069799

(56) Entgegenhaltungen:
- US-B1- 6 359 139
- DEUTSCH H M ET AL: "SYNTHESIS AND PHARMAMOLOGY OF POTENTIAL COCAINE ANTAGONISTS. 2. STRUCTURE-ACTIVITY RELATIONSHIP STUDIES OF AROMATIC RING- SUBSTITUTED METHYLPHENIDATE ANALOGS" JOURNAL OF MEDICINAL CHEMISTRY, AMERICAN CHEMICAL SOCIETY. WASHINGTON, US, Bd. 39, 15. März 1996 (1996-03-15), Seiten 1201-1209, XP000602079 ISSN: 0022-2623
- PAQUETTE L. A. ET AL: "Ring and C-O Bond Fragmentation as Tools for Fingerprinting the Extent of Homolysis during Base-Catalyzed Carbon-Carbon Bond Cleavages of the Haller-Bauer, Cram, and Gilday Types" JOURNAL OF ORGANIC CHEMISTRY, Bd. 54, Nr. 21, 1989, Seiten 5054-5063, XP002253202
- MOLL F.: "Kondensierte Azetidinone-(2)" ARCHIV DER PHARMAZIE UND BERICHTE DER DEUTSCHEN PHARMAZEUTISCHEN GESELLSCHAFT, Bd. 301, Nr. 4, 1968, Seiten 250-262, XP008021216

## Beschreibung

Die vorliegende Erfindung betrifft Verfahren zur Herstellung von d-threo-2-Phenyl-2-piperidin-2-yl-essigsäureestern und deren pharmazeutisch annehmbaren Säureadditionssalzen, insbesondere Verfahren zur Herstellung des d-threo-[R(R*,R*)]-2-Phenyl-2-piperidin-2-yl-essigsäuremethylester-Hydrochlorids (d-threo-Methylphenidat-Hydrochlorid). D-threo-2-Phenyl-2-piperidin-2-yl-essigsäureester und deren Säureadditionssalze sind an sich bekannt. Insbesondere d-threo-Methylphenidat-Hydrochlorid ist eine bekannte pharmazeutisch wirksame Verbindungen.

D-threo-Methylphenidat und entsprechende Säureadditionssalze sowie deren Herstellung sind aus US 2,507,631, US 2,957,880 Sowie US635919 und J. Med. Chem. 1996, 39, 1201-1209 bekannt. EP-A-0 948 484 beschreibt die Anreicherung von threo-Methylphenidat und entsprechender Säureadditionssalze in enantiomeren Gemischen derselben, unter Verwendung von Kristallisationsverfahren.

Es besteht immer noch Bedarf an der Verbesserung des Herstellungsverfahrens, insbesondere für d-threo-Methylphenidat-Hydrochlorid, um die Ausbeute und die optische Reinheit sowie auch die Wirtschaflichkeit des Produktes zu verbessern.

Es wurde nun gefunden, dass sich d-threo-[R(R*,R*)]-2-Phenyl-2-piperidin-2-yl-essigsäureester sowie die entsprechenden Säureadditionssalze, insbesondere d-threo-Methylphenidat-Hydrochlorid, aus d-threo-[R(R*,R*)]-2-Phenyl-2-piperidin-2-yl-essigsäure herstellen lassen, ohne dass die stereospezifischen Merkmale verloren gehen, wenn man d-threo-[R(R*,R*)]-2-Phenyl-2-piperidin-2-yl-essigsäure zuerst in das entsprechende d-threo-[R(R*,R*)]-2-Phenyl-2-piperidin-2-yl-essigsäurehalogenid, vorzugsweise in das d-threo-[R(R*,R*)]-2-Phenyl-2-piperidin-2-yl-essigsäurechlorid, umwandelt, und anschliessend aus diesem den Ester herstellt.

Die vorliegende Erfindung ist in den Patentansprüchen definiert. Insbesondere betrifft die vorliegende Erfindung Verfahren zur Herstellung von d-threo-[R(R*,R*)]-2-Phenyl-2-piperidin-2-yl-essigsäureester und deren Säureadditionssalze, welches dadurch gekennzeichnet ist, dass man d-threo-[R(R*,R*)]-2-Phenyl-2-piperidin-2-yl-essigsäure oder ein Säureadditionssalz davon in das entsprechende d-threo-[R(R*,R*)]-2-Phenyl-2-piperidin-2-yl-essigsäurehalogenid, vorzugsweise in das d-threo-[R(R*,R*)]-2-Phenyl-2-piperidin-2-yl-essigsäurechlorid, umwandelt, aus diesem den gewünschten d-threo-[R(R*,R*)]-2-Phenyl-2-piperidin-2-yl-essigsäureester oder ein Säureadditionssalz davon durch Veresterung herstellt und das erhaltene Produkt gegebenenfalls umkristallisiert.

Als Ester kommen (C₁₋₄)-Alkylester in Frage, insbesondere der Methylester. Vorzugsweise stellt man d-threo-Methylphenidat-Hydrochlorid her.

Additionssalze sind beispielsweise die Salze von Salzsäure, Bromwasserstoff, von Weinsäure und Weinsäurederivaten oder andern verwandten organischen Säuren. Bevorzugt ist das Hydrochlorid.

Die Bedingungen für die Halogenierung von D-threo-[R(R*,R*)]-2-Phenyl-2-piperidin-2-yl-essigsäure zur Herstellung des entsprechenden Halogenids sind aus analogen Reaktionen bekannt. Bevorzugt ist das d-threo-[R(R*,R*)]-2-Phenyl-2-piperidin-2-yl-essigsäurechlorid, welches durch Chlorierung der entsprechenden d-threo-[R(R*,R*)]-2-Phenyl-2-piperidin-2-yl-essigsäure, vorzugsweise mittels Thionylchlorid, in an sich bekannter Weise erhalten werden kann. Das derart erhaltene Säurechlorid wird dann mit wasserfreiem Alkohol, vorzugsweise wasserfreiem Methanol, in den gewünschten d-threo-[R(R*,R*)]-2-Phenyl-2-piperidin-2-yl-essigsäureester, vorzugsweise in d-threo-Methylphenidat, unter Abspaltung von Halogenwasserstoff, umgewandelt. Das erhaltene Produkt kann durch Kristallisation gereinigt werden. Dabei erhält man ein Produkt, welches einen enantiomeren Überschuss [enantiomeric excess (ee)] an d-threo-[R(R*,R*)]-2-Phenyl-2-piperidin-2-yl-essigsäureester von mindestens 98.% (ee> 98.%) aufweist.

Die Halogenierungsreaktion wird vorzugsweise im Temperaturbereich von unter 90°C, vorzugsweise zwischen 70°C bis 80°C, durchgeführt, wobei nach beendeter Halogenierung und Entfernung der flüchtigen Bestandteile sofort verestert wird.

Für die Veresterungsreaktion mit Alkohol geht man vorzugsweise so vor, dass man dem Reaktionsgemisch, aus welchem vorgehend die flüchtigen Bestandteile durch Erhitzen im Vakuum entfernt worden sind, den Alkohol zusetzt. Dabei ist eine Reaktionstemperatur im Bereich von 35°C bis 50°C bevorzugt. Anschliessend wird der überschüssige Alkohol im Vakuum entfernt, um die Isolation des Reaktionsproduktes zu erleichtern, da dieses in der Regel gut in Alkohol löslich ist.

Die vorliegende Erfindung betrifft im weiteren auch ein Verfahren zur Umkristallisation von d-threo-Methylphenidat-Hydrochlorid, welches dadurch gekennzeichnet ist, dass man rohes d-threo-Methylphenidat-Hydrochlorid in Wasser auf eine Temperatur von 80°C und vorzugsweise auf 50°C bis 70°C erhitzt bzw. löst, die Lösung gegebenenfalls von allfälligen Rückständen filtriert, anschliessend die Lösung auf Raumtemperatur (15-25°C) abkühlt und dann gasförmigen Chlorwasserstoff bis zu einem Gehalt im Bereich von 12 Gew.-% bis 20 Gew.-%, einleitet, worauf reines d-threo-Methylphenidat-Hydrochlorid ausfällt und abfiltriert werden kann. Die optische Reinheit beträgt in der Regel ee>98%. Überraschend ist, dass bei diesem Rekristallisationsverfahren allfällig vorhandenes erythro-Isomer entfernt wird. Organische Lösungsmittel müssen nicht verwendet werden.

D-threo-[R(R*,R*)]-2-Phenyl-2-piperidin-2-yl-essigsäure, stellt man her, indem man d-threo-[R(R*,R*)]-2-Phenyl-2-piperidin-2-yl-essigsäureamid mit einer geeigneten Säure, vorzugsweise Salzsäure, hydrolysiert.

Im weiteren wird ein Verfahren zur Herstellung von reinem D-threo-[R(R*,R*)]-2-Phenyl-2-piperidin-2-yl-essigsäureamid beschrieben, welches als Ausgangsmaterial für die Herstellung der entsprechenden Carbonsäure eingesetzt wird. Die Verwendung von reinem D-threo-[R(R*,R*)]-2-Phenyl-2-piperidin-2-yl-essigsäureamid hat den überraschenden Vorteil, dass das Endprodukt in chemisch und optisch sehr reiner Form erhalten wird. Zudem kann bereits auf der Stufe der Amidhydrolyse mit erheblich kleineren Stoffmengen gearbeitet werden, was auch deutliche wirtschaftliche Vorteile mit sich bringt.

Man stellt das zu verwendende reine d-threo-[R(R*,R*)]-2-Phenyl-2-piperidin-2-yl-essigsäureamid her, indem man ein racemisches Gemisch von 2-Phenyl-2-piperidin-2-yl-essigsäureamid, welches d-threo-[R(R*,R*)]-2-Phenyl-2-piperidin-2-yl-essigsäureamid enthält, mit einem Weinsäurederivat in einem geeigneten Lösungsmittel, vorzugsweise in Isopropanol, umsetzt, wobei d-threo-[R(R*,R*)]-2-Phenyl-2-piperidin-2-yl-essigsäureamid-D-tartrat erhalten wird, welches bei einer Temperatur vorzugsweise im Bereich von 25-40°c auskristallisiert und aus dem Gemisch abgetrennt wird.

Das derart erhaltene d-threo-[R(R*,R*)]-2-Phenyl-2-piperidin-2-yl-essigsäureamid-dibenzoyl-D-tartrat entspricht der chemischen Formel:

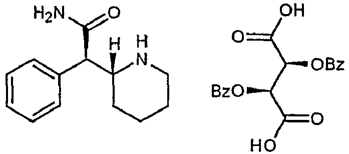

Das abgetrennte d-threo-[R(R*,R*)]-2-Phenyl-2-piperidin-2-yl-essigsäureamid-dibenzoyl-D-tartrat wird vorzugsweise durch Kristallisation gereinigt. Dabei wird bis zur Lösung der Verbindung im Lösungsmittel, vorzugsweise in Isopropanol, erhitzt, wobei genügend Lösungsmittel anwesend ist. Man erhitzt vorzugsweise bis auf etwa 70°C und kühlt anschliessend langsam bis auf eine Temperatur von 25°C ab. Vorzugsweise wird im Temperaturbereich von 25-37°C, vorzugsweise bei 25-29°C auskristallisiert. Dabei werden optische Reinheiten von ee>98% erhalten. Bei Temperaturen unter 25°C kristallisiert das Erythro-Isomer aus.

Aus dem d-threo-[R(R*,R*)]-2-Phenyl-2-piperidin-2-yl-essigsäureamid-dibenzoyl-D-tartrat kann nun das d-threo-[R(R*,R*)]-2-Phenyl-2-piperidin-2-yl-essigsäureamid mittels Behandlung mit Alkali, vorzugsweise mit Natriumhydroxid, gewonnen werden. Dabei behandelt man d-threo-[R(R*,R*)]-2-Phenyl-2-piperidin-2-yl-essigsäureamid-dibenzoyl-D-tartrat vorzugsweise mit einer etwa 30%-igen wässerigen Natriumhydroxidlösung bei Raumtemperatur, d.h. bei 15-25°C, und kühlt anschliessend auf eine Temperatur vorzugsweise im Bereich von 2-10°C, worauf das d-threo-[R(R*,R*)]-2-Phenyl-2-piperidin-2-yl-essigsäureamid auskristallisiert und abfiltriert werden kann.

### Referenz-Beispiel 1: (Herstellung von racemischen threo-2-Phenyl-2-piperidin-2-yl-essigsäureamid)

In einem 250 ml Rundkolben werden 91.6 g einer 30%igen wässerigen Natriumhydroxidlösung mit 53 g Wasser verdünnt. Hierauf fügt man unter Rühren bei Raumtemperatur eine racemische Mischung von erythro/threo-2-Phenyl-2-piperidin-2-yl-essigsäureamid hinzu und rührt weitere 5 bis 10 Minuten bis eine gleichmässige Suspension erhalten wird. Hierauf erhitzt langsam man auf 101-103°C und hält diese Temperatur weitere 5 Stunden aufrecht. Die Temperatur von 108°C sollte nicht überschritten werden. Die erhaltene Aufschlämmung wird anschliessend schnell auf 20±5°C abgekühlt und über Nacht gerührt. Die Aufschlämmung wird zentrifugiert und das erhaltene Zentrifugat sorgfältig mit Wasser gewaschen, und im Vakuum bei <200 mbar und einer Temperatur von 55±5°C getrocknet. Man erhält eine Ausbeute von 29.7g (90%) mit einem Threo-Gehalt von de ~85% (ermittelt mit HPLC).

### Referenz-Beispiel 2: (Herstellung von d-threo-[R(R*,R*)]-2-Phenyl-2-piperidin-2-yl-essigsäureamid-dibenzoyl-D-tartrat)

In einem 500 ml Rundkolben werden bei Raumtemperatur 333.1 g Isopropanol sowie 16.1 g der vorgehend in Beispiel 1 hergestellten racemischen Mischung enthaltend threo-2-Phenyl-2-piperidin-2-yl-essigsäureamid (de ~85%) und 26.5 g Dibenzoyl-D-Weinsäure vorgelegt und gerührt bis eine gleichmässige weisse Suspension erhalten wird, worauf die Suspension auf 55±5°C erhitzt, eine weitere Stunde bei dieser Temperatur gehalten und langsam auf 37°C abgekühlt wird. Die Temperatur von 37°C wird während 90 Minuten gehalten, worauf weiter auf 27°C abgekühlt wird. Die Temperatur von 27°C wird nun während etwa 4 Stunden gehalten, bis alles d-threo-[R(R*,R*)]-2-Phenyl-2-piperidin-2-yl-essigsäureamid-dibenzoyl-D-tartrat auskristallisiert ist. Die Temperatur soll nicht unter 25°C fallen. Die erhaltene Suspension wird filtriert und der Filterkuchen mit Isopropanol bei einer Temperatur von 20-25°C gewaschen. Darauf wird der feuchte Filterkuchen bei 45-55°C im Vakuum bei einem Druck von <200 mbar über Nacht getrocknet. Man erhält eine Ausbeute an d-threo-[R(R*,R*)]-2-Phenyl-2-piperidin-2-yl-essigsäureamid-dibenzoyl-D-tartrat von 88.0% berechnet auf d-threo-[R(R*,R*)]-2-Phenyl-2-piperidin-2-yl-essigsäureamid, in einer Reinheit von 99.5% (ermittelt mit HPLC).

### Referenz-Beispiel 3: (Herstellung von d-threo-[R(R*,R*)]-2-Phenyl-2-piperidin-2-yl-essigsäureamid)

In einem 100ml Rundkolben werden 27.9 g Wasser und 12.8 g einer 30%-igen wässrigen Lösung von Natriumhydroxid vorgelegt. Unter Rühren werden langsam 17.26 g des in Beispiel 2 hergestellten d-threo-[R(R*,R*)]-2-Phenyl-2-piperidin-2-yl-essigsäureamid-dibenzoyl-D-tartrat während etwa 60 Minuten bei einer Temperatur im Bereich von 15-25°C zugegeben, wobei in einer exothermen Reaktion d-threo-[R(R*,R*)]-2-Phenyl-2-piperidin-2-yl-essigsäureamid freigesetzt wird. Die erhaltene Suspension wird nun unter Rühren auf 4-10°C abgekühlt und bei dieser Temperatur während etwa 60 Minuten gehalten. Die Suspension wird dann bei 4-10°C zentrifugiert. Die erhaltenen Kristalle werden mit verdünnter NaOH und dann mit Wasser gewaschen und anschliessend im Vakuum bei 45-55°C über Nacht getrocknet. Die Ausbeute an reinem d-threo-[R(R*,R*)]-2-Phenyl-2-piperidin-2-yl-essigsäureamid (ee∼98%) beträgt ca. 80%.

### Beispiel 4: (Herstellung von d-threo-[R(R*,R*)]-2-Phenyl-2-piperidin-2-yl-essigsäure-Hydrochlorid)

In einem 100ml Rundkolben werden 6.36 g Wasser und 17.34 g einer 32%-igen wässrigen Chlorwasserstoff-Lösung (Salzsäure) vorgelegt. Unter Rühren werden 5.14 g des in Beispiel 3 hergestellten d-threo-[R(R*,R*)]-2-Phenyl-2-piperidin-2-yl-essigsäureamids bei Raumtemperatur zugegeben, wobei in eine leicht exotherme Reaktion auftritt. Die erhaltene Mischung wird nun unter Rühren innerhalb etwa einer Stunde auf 100-107°C erhitzt und bei dieser Temperatur während etwa 5 Stunden unter Rühren gehalten. Die Mischung wird dann innerhalb etwa einer Stunde auf einen Temperaturbereich von -3°C bis 1°C abgekühlt, worauf die Suspension zentrifugiert wird. Die erhaltenen Kristalle werden mit Wasser gewaschen und anschliessend im Vakuum bei 45-55°C über Nacht getrocknet. Die Ausbeute an d-threo-[R(R*,R*)]-2-Phenyl-2-piperidin-2-yl-essigsäure-Hydrochlorid beträgt 4.7 g bei einer Reinheit (ee) von 95% (bestimmt mit HPLC).

### Beispiel 5: (Herstellung von d-threo-Methylphenidat-Hydrochlorid)

a) In einem 250ml Rundkolben werden 26.0 g Toluol, 0.02 g N,N-Dimethylformamid und 2.6 g des gemäss Beispiel 4 hergestellten d-threo-[R(R*,R*)]-2-Phenyl-2-piperidin-2-yl-essigsäure-Hydrochlorids vorgelegt. Hierauf werden unter Rühren bei Raumtemperatur 3.6 g Thionylchlorid während etwa 15 Minuten bei Raumtemperatur zugesetzt. Anschliessend wird die Mischung während etwa 30 Minuten auf etwa 82°C erhitzt und bei dieser Temperatur während weiteren zwei Stunden gehalten. Dabei entwickelt sich Chlorwasserstoff (HCl) und Schwefeldioxid (SO₂), welche in einem mit NaOH beladenem Absorber absorbiert werden. Das Reaktionsgemisch wird anschliessend auf 45-55°C abgekühlt und mittels HPLC analysiert. Die Reaktion wird fortgesetzt, bis der Gehalt an Säurechlorid mindestens 95% beträgt. Das Reaktionsgemisch wird dann unter Vakuum (90-130 mbar) bei einer Temperatur im Bereich von 37-53°C gesetzt, um alle flüchtigen Reaktionskomponenten zu entfernen, was etwa nach einer Stunde der Fall ist.
b) Das unter Abschnitt a) erhaltene Reaktionsgemisch wird nun auf eine Temperatur im Bereich von 37-43°C abgekühlt und langsam und unter Rühren zu 6.2 g Methanol zugegeben. Es erfolgt eine exotherme Reaktion, so dass das Reaktionsgemisch gekühlt werden muss, um es bei einer Temperatur von 37-43°C zu halten. Nach erfolgter Zugabe wird das Reaktionsgemisch noch etwa 2 Stunden im Temperaturbereich von 37-50°C gehalten und gerührt. Das Reaktionsgemisch wird anschliessend mittels HPLC analysiert und die Reaktion fortgesetzt, bis der Gehalt an d-threo-Methylphenidat mindestens 95% beträgt. Das Reaktionsgemisch wird nun bei einer Temperatur im Bereich von 25-45°C unter Vakuum (90-130 mbar) gesetzt, um alle flüchtigen Reaktionskomponenten zu entfernen, was etwa nach einer Stunde der Fall ist. Die Mischung wird nun langsam auf 10-20°C abgekühlt, worauf die Suspension zentrifugiert wird. Die erhaltenen Kristalle werden mit Toluol gewaschen und anschliessend im Vakuum bei 45-55°C über Nacht getrocknet. Die Ausbeute an d-threo-Methylphenidat-Hydrochlorid (roh) beträgt 2.5 g bei einer Reinheit (ee) von 95% (bestimmt mit HPLC).

### Beispiel 6: (Reinigung von rohem d-threo-Methylphenidat-Hydrochlorid)

In einem 50 ml Rundkolben werden 6.5 g deionisiertes Wasser sowie 2.42 g des in Beispiel 5 hergestellten d-threo-Methylphenidat-Hydrochlorid (roh) und 0.05 g Entfärbungskohle während 30 Minuten unter Rühren auf 60-70°C erwärmt und weitere 30 Minuten bei dieser Temperatur gehalten. Dann wird filtriert und bei 60°C mit deionisiertem Wasser von 60°C nachgewaschen. Die erhaltene klare Lösung wird auf eine Temperatur im Bereich von 15-25°C abgekühlt, worauf wasserfreies Chlorwasserstoffgas direkt in die Lösung eingeleitet wird bis zu einer Konzentration von etwa 18 Gew.-%. Dabei soll die Temperatur der Lösung 35°C nicht übersteigen. Die erhaltene Suspension wird auf 0-6°C gekühlt und bei dieser Temperatur während 90 Minuten gerührt, worauf die Suspension bei einer Temperatur im Bereich von 0-6°C zentrifugiert wird. Die erhaltenen Kristalle werden mit kaltem Isopropanol gewaschen und anschliessend im Vakuum bei 45-55°C über Nacht getrocknet. Die Ausbeute an reinem d-threo-Methylphenidat-Hydrochlorid (bezogen auf das rohe Ausgangsprodukt) beträgt 73% bei einer Reinheit (ee) von >99% (bestimmt mit HPLC).

## Patentansprüche

1. Verfahren zur Herstellung von d-threo-[R(R*,R*)]-2-Phenyl-2-piperidin-2-yl-essigsäureester und deren Säureadditionssalze, **dadurch gekennzeichnet, dass** man d-threo-[R(R*,R*)]-2-Phenyl-2-piperidin-2-yl-essigsäure oder ein Säureadditionssalz davon in das entsprechende d-threo-[R(R*,R*)]-2-Phenyl-2-piperidin-2-yl-essigsäurehalogenid umwandelt, aus diesem den gewünschten d-threo-[R(R*,R*)]-2-Phenyl-2-piperidin-2-yl-essigsäureester oder ein Säureadditionssalz davon durch Veresterung herstellt, und das erhaltene Produkt gegebenenfalls umkristallisiert.

2. Verfahren nach Anspruch 1, **dadurch gekennzeichnet, dass** man als d-threo-[R(R*,R*)]-2-Phenyl-2-piperidin-2-yl-essigsäure-halogenid das d-threo-[R(R*,R*)]-2-Phenyl-2-piperidin-2-yl-essig-säurechlorid herstellt.

3. Verfahren nach Anspruch 1 oder 2, **dadurch gekennzeichnet, dass** man als d-threo-[R(R*,R*)]-2-Phenyl-2-piperidin-2-yl-essigsäureester einen (C₁₋₄)-Alkylester, vorzugsweise den Methylester, vorzugsweise d-threo-Methylphenidat-Hydrochlorid herstellt.

4. Verfahren nach Anspruch 1 oder 2, **dadurch gekennzeichnet, dass** man d-threo-[R(R*,R*)]-2-Phenyl-2-piperidin-2-yl-essigsäure-Hydrochlorid mit Thionylchlorid in einem inerten Lösungsmittel, vorzugsweise in Toluol, und in Gegenwart eines katalytisch wirkenden aprotischen Lösungsmittels, vorzugsweise Dimethylformamid, chloriert, und anschliessend das erhaltene Säurechlorid mit wasserfreiem Alkohol, vorzugsweise wasserfreiem Methanol, in den gewünschten d-threo-[R(R*,R*)]-2-Phenyl-2-piperidin-2-yl-essigsäureester unter Abspaltung von Halogenwasserstoff umwandelt.

5. Verfahren nach Anspruch 4, **dadurch gekennzeichnet, dass** man die Halogenierung im Temperaturbereich von unter 90°C, vorzugsweise zwischen 70°C bis 80°C, durchführt.

6. Verfahren zum Umkristallisieren von d-threo-Methylphenidat-Hydrochlorid nach Anspruch 1, **dadurch gekennzeichnet, dass** man d-threo-Methylphenidat-Hydrochlorid in Wasser auf eine Temperatur von 80°C und vorzugsweise auf 50°C bis 70°C erhitzt bzw. löst, anschliessend die Lösung auf Raumtemperatur abkühlt und gasförmigen Chlorwasserstoff bis zu einem Gehalt im Bereich von 12 Gew.-% bis 20 Gew.-%, einleitet und das als Niederschlag erhaltene reine d-threo-Methylphenidat-Hydrochlorid abfiltriert.

7. Verfahren nach Anspruch 1, **dadurch gekennzeichnet, dass** die verwendete d-threo-[R(R*,R*)]-2-Phenyl-2-piperidin-2-yl-essigsäure aus d-threo-[R(R*,R*)]-2-Phenyl-2-piperidin-2-yl-essigsäureamid durch Hydrolyse mit einer geeigneten Säure, vorzugsweise durch Hydrolyse mit Salzsäure, hergestellt wurde.

## Claims

1. A method for synthesis of d-threo-[R(R*,R*)]-2-phenyl-2-piperidin-2-yl acetic acid ester and its acid addition salts, **characterized in that** d-threo-[R(R*,R*)]-2-phenyl-2-piperidin-2-yl acetic acid or an acid addition salt thereof is converted to the corresponding d-threo-[R(R*,R*)]-2-phenyl-2-piperidin-2-yl acetic acid halide from which the desired d-threo-[R(R*,R*)]-2-phenyl-2-piperidin-2-yl acetic acid ester or an acid addition salt thereof is synthesized by esterification and the resulting product is optionally recrystallized.

2. The method according to claim 1, **characterized in that** d-threo-[R(R*,R*)]-2-phenyl-2-piperidin-2-yl acetic acid chloride is synthesized as the d-threo-[R(R*,R*)]-2-phenyl-2-piperidin-2-yl acetic acid halide.

3. The method according to claim 1 or 2, **characterized in that** a C₁₋₄ alkyl ester, preferably the methyl ester, preferably d-threo-methylphenidate hydrochloride is synthesized as the d-threo-[R(R*,R*)]-2-phenyl-2-piperidin-2-yl acetic acid ester.

4. The method according to claim 1 or 2, **characterized in that** d-threo-[R(R*,R*)]-2-phenyl-2-piperidin-2-yl acetic acid chloride is chlorinated with thionyl chloride in an inert solvent, preferably in toluene and in the presence of a catalytic aprotic solvent, preferably dimethyl formamide, and then the resulting acid chloride is converted with anhydrous alcohol, preferably anhydrous methanol, into the desired d-threo-[R(R*,R*)]-2-phenyl-2-piperidin-2-yl acetic acid ester, splitting off hydrogen halide.

5. The method according to claim 4, **characterized in that** the halogenation is performed in the temperature range of less than 90°C, preferably between 70°C and 80°C.

6. The method for recrystallizing d-threo-methylphenidate hydrochloride according to claim 1, **characterized in that** d-threo-methylphenidate hydrochloride is heated in water to a temperature of 80°C and preferably to 50°C to 70°C and/or is dissolved, then the solution is cooled to room temperature and hydrogen chloride gas is introduced up to a content in the range of 12 wt% to 20 wt%, and the pure d-threo-methylphenidate hydrochloride which is obtained as a precipitate is filtered out.

7. The method according to claim 1, **characterized in that** the d-threo-[R(R*,R*)]-2-phenyl-2-piperidin-2-yl acetic acid that is used was synthesized from d-threo-[R(R*,R*)]-2-phenyl-2-piperidin-2-yl acetic acid amide by hydrolysis with a suitable acid, preferably by hydrolysis with hydrochloric acid.

## Revendications

1. Procédé de fabrication d'esters d'acide d-thréo-[R(R*,R*)]-2-phényl-2-pipéridin-2-yl-acétique et de leurs sels d'addition avec les acides, **caractérisé par le fait que** l'on transforme de l'acide d-thréo-[R(R*,R*)]-2-phényl-2-pipéridin-2-yl-acétique ou un sel d'addition avec un acide de celui-ci en l'halogénure d'acide d-thréo-[R(R*,R*)]-2-phényl-2-pipéridin-2-yl-acétique correspondant, à partir duquel on prépare par estérification l'ester d'acide d-thréo-[R(R*,R*)]-2-phényl-2-pipéridin-2-yl-acétique souhaité ou un sel d'addition avec un acide de celui-ci et on recrisallise le cas échéant le produit obtenu.

2. Procédé selon la revendication 1, **caractérisé par le fait que** l'on prépare, comme halogénure d'acide d-thréo-[R(R*,R*)]-2-phényl-2-pipéridin-2-yl-acétique, le chlorure d'acide d-thréo-[R(R*,R*)]-2-phényl-2-pipéridin-2-yl-acétique.

3. Procédé selon l'une des revendications 1 ou 2, **caractérisé par le fait que** l'on prépare, comme ester d'acide d-thréo-[R(R*,R*)]-2-phényl-2-pipéridin-2-yl-acétique, un (alkyl en C₁₋₄)ester, de préférence l'ester méthylique, de préférence le chlorhydrate de d-thréo-méthylphénidate.

4. Procédé selon l'une des revendications 1 ou 2, **caractérisé par le fait que** l'on chlore du chlorhydrate d'acide d-thréo-[R(R*,R*)]-2-phényl-2-pipéridin-2-yl-acétique par du chlorure de thionyle dans un solvant inerte, de préférence dans le toluène, et en présence d'un solvant aprotique à action catalytique, de préférence le diméthylformamide, puis l'on transforme le chlorure d'acide obtenu avec de l'alcool anhydre, de préférence du méthanol anhydre, en l'ester d'acide d-thréo-[R(R*,R*)]-2-phényl-2-pipéridin-2-yl-acétique souhaité, avec élimination d'halogénure d'hydrogène.

5. Procédé selon la revendication 4, **caractérisé par le fait que** l'on conduit l'halogénation dans la plage de température de moins de 90°C, de préférence entre 70°C et 80°C.

6. Procédé de recristallisation de chlorhydrate de d-thréo-méthylphénidate selon la revendication 1, **caractérisé par le fait que** l'on chauffe ou l'on dissout du chlorhydrate de d-thréo-méthylphénidate dans de l'eau à une température de 80°C et, de préférence, à 50°C à 70°C, puis l'on refroidit la solution jusqu'à la température ambiante et l'on y introduit du chlorure d'hydrogène gazeux jusqu'à une teneur se situant dans la plage de 12 % en poids jusqu'à 20 % en poids et l'on sépare par filtration le chlorhydrate de d-thréo-méthylphénidate pur obtenu en tant que précipité.

7. Procédé selon la revendication 1, **caractérisé par le fait que** l'acide d-thréo-[R(R*,R*)]-2-phényl-2-pipéridin-2-yl-acétique utilisé a été préparé à partir d'amide d'acide d-thréo-[R(R*,R*)]-2-phényl-2-pipéridin-2-yl-acétique par hydrolyse avec un acide approprié, de préférence par hydrolyse avec de l'acide chlorhydrique.
